(19) Europäisches Patentamt

European Patent Office

Office européen des brevets

(11) **EP 1 298 432 A1**

(12) **EUROPEAN PATENT APPLICATION**

(43) Date of publication:
02.04.2003 Bulletin 2003/14

(51) Int Cl.$^7$: **G01N 33/38**

(21) Application number: **01402502.7**

(22) Date of filing: **28.09.2001**

(84) Designated Contracting States:
**AT BE CH CY DE DK ES FI FR GB GR IE IT LI LU
MC NL PT SE TR**
Designated Extension States:
**AL LT LV MK RO SI**

(71) Applicants:
- **SERVICES PETROLIERS SCHLUMBERGER
  75007 Paris (FR)**
  Designated Contracting States:
  **FR**
- **Schlumberger Technology B.V.
  2514 JG Den Haag (NL)**
  Designated Contracting States:
  **AT DE DK IT**
- **SCHLUMBERGER HOLDINGS LIMITED
  Road Town, Tortola (VG)**
  Designated Contracting States:
  **GB NL**
- **SOFITECH N.V.
  1180 Bruxelles (BE)**
  Designated Contracting States:
  **BE CH LI CY ES FI GR IE LU MC PT SE TR**

(72) Inventors:
- **Barlet-Gouedard, Véronique
  92290 Chatenay Malabry (FR)**
- **Thiercelin, Marc
  92410 Ville d'Avray (FR)**
- **Vigneaux, Pierre
  77950 Moisenay (FR)**

(74) Representative: **Hyden, Martin
  Etudes et Productions Schlumberger,
  Intellectual Property Department,
  1, rue Henri Becquerel
  92142 Clamart Cedex (FR)**

(54) **Methods for characterising gas-tight cement slurries**

(57)    A method of characterising the gas migration in a cement slurry, comprises measuring the shear modulus of the slurry over a period of time and using the measurement of the shear modulus to characterise the gas migration in the slurry.

**EP 1 298 432 A1**

**Description**

The present invention relates to methods of determining the gas migration control properties of a cement slurry for use in oil well cementing or the like. The invention also comprises methods of designing gas-tight cement slurries and the slurries produced to such designs.

Annular gas migration may occur during drilling or well completion procedures and has been recognised as one of the most troublesome problems of the petroleum industry. Annular gas migration consists of the invasion of formation gas into the annulus. During cement placement, the slurry will develop gel strength. Due to its capability to sustain shear stresses, hydrostatic pressure within the annulus is continually reduced by slurry gelation, leading to the possibility that gas can flow to a lower pressure zone or to the surface. Gel strength development has been a known as a problem for gas migration since 1973.

Primary Cementing is the process of placing cement in the annulus between the casing and the formations exposed to the wellbore. Casings are cemented to prevent drilling fluid from circulating outside of the casing, seal off abnormal pressure formations or protect casings from any corrosive fluids existing in subsurface formations. The major objective of cementing is to provide zonal isolation in the wellbore of oil, gas and water wells. To achieve this objective, a hydraulic seal must be obtained between the casing and the cement and between the casing and the formations while at the same time preventing fluid channels in the cement sheath.

The basic process for accomplishing a primary cement job is as follows:
After drilling the well to the desired position, the drillpipe is removed and a larger string of casing is run into the well until it reaches the bottom of the well. At this time the drilling mud, which is used to remove the cuttings and provide a hydrostatic head on the well, is still in the wellbore. This mud must be removed and replaced by the hardened cement. The process to accomplish this is the two plug cementing method. Two plugs are used to isolate the cement as it is pumped down the casing to prevent contamination with the mud. Sufficient cement is pumped into the casing to fill the annular column from the bottom up to at least across the productive zones or the zones to be isolated. The cementing process is completed when a pressure increase at the surface indicates the top plug has reached the landing collar or float collar at the bottom of the casing, and displacement with mud or water is terminated. The well is shut in to allow the cement to harden before beginning completion work or drilling out to a deeper horizon.

For most oil well cementing jobs, Portland cement is used. The conditions in a well differ significantly from those encountered at ambient conditions during construction operations. Portland cement is especially made for use in oil or gas wells. Portland cement is hydraulic cement which means that as the cement sets, it develops compressive strength as a result of hydration, which involves chemical reaction between water and the compounds present in the cement, not upon a drying- out process.

Portland cement consists of four compounds: tricalcium silicate ($Ca_3SiO_5$); dicalcium silicate ($Ca_2SiO_4$); tricalcium aluminaat ($Ca_3Al_2O_6$); and tetracalcium aluminoferrite ($Ca_4Al_2Fe_2O_6$) These compounds are formed in a kiln by a series of reactions at temperatures as high as $1500°$ C between lime, silica, alumina and iron oxide. In the manufacturing process selected raw materials are ground to a fine powder, and proportioned in such a way that the resulting mixture has a desired chemical composition. After blending, the raw material mixture is fed into a kiln and converted to cement clinker. The clinker is cooled, a small amount of gypsum (3-5%) is added and the mixture is pulverized. The pulverized product is finished Portland cement.

Portland cements are manufactured to meet certain physical and chemical standards, which depend upon their application. To promote consistency of performance among cement manufactures, classification systems and specifications have been established by various user groups, for example the system of American Petroleum Institute (API). There are currently eight classes of API Portland cements (A- G), arranged according to the depth, temperature and pressure of use. Classes G and H are the most commonly used well cements today, intended for use as basic well cements from surface to 8000 ft (2440 m) depth as manufactured, or with additives (accelerators and retarders) to cover a wide range of well depths and temperatures.

The silica phases in Portland cement are the most abundant ($Ca_2SiO_4$ and $Ca_3SiO_5$). $Ca_3SiO_5$ is the principal constituent, with a concentration as high as 70%. The equation below shows the idealized situation. The hydration products for both phases are calcium silicate hydrate and calcium hydroxide.

$$2(Ca_3SiO_5) + 6(H_2O) \rightarrow Ca_3Si_2H_6O_{10} + Ca_3(H_2O_2)_3$$

The calcium - silicate- hydrate $Ca_3Si_2H_6O_{14}$ also called C-S-H gel, is largely amorphous and comprises roughly 70% of the set cement and gives cement it's strength. At first, these hydration reactions proceed vigorously and a dense layer of C-S-H gel builds up around each silicate particle. But the gel is relatively impermeable and it soon prevents more water from reaching the surface of the anhydrous silicates, hindering further hydration. An interval of low reactivity follows and is this is called the induction period. Hydration eventually picks up when the permeability of the C-S-H gel layer begins increasing, allowing more water to reach the silicate grain surface.

The hydration of Portland cement can be considered a sequence of overlapping reactions leading to a continuous thickening and hardening cement slurry. During initial hydration, when the anhydrous material is added to water and hydration products begin to form, the cement grains stay independent and can still be pumped. When hydration picks up after the induction period, the cement grains begin to link together and the slurry is not pumpable anymore. The development of additives can change this behaviour of the slurry.

Examples of the various additives are shown below:

| Accelerator | shorter thickening time, higher early compressive strength | $CaCl_2$, NaCl |
| Retarder | longer thickening time | lignosulfonates, cellulose derivatives |
| Extender | lower slurry density higher slurry yield | Bentonite, Sodium silicates, Pozzolans, Gilsonite, Powdered coal, Microspheres, Nitrogen |
| Weighting agent | Higher slurry density | Barite ($BaSO_4$), Hematite ($Fe_2O_3$), Ilmenite ($FeTiO_3$) |
| Dispersant | Lower slurry viscosity | polynaphtalene sulfonate, lignosulfonates, hydroxylated polysaccharides |
| fluid-loss additive | Reduced slurry dehydration | Cellulosic polymers, Polyamines, Bentonite, Polyvinylalcohol, Latices |
| lost-circulation control agent | Prevent loss of slurry to formation | Gilsonite, Granular coal, Gypsum, Bentonite |
| Antifoam agent | Reduced air entrainment aid for slurry mixing | Polyglycol ethers, Silicones |
| Strengthening agent | | Nylon fibers, Ground rubber |

Annular gas migration may occur during drilling or well completion procedures and has been recognised as one of the most troublesome problems of the petroleum industry. Annular gas migration consists of the invasion of formation gas into the annulus. Because of a pressure imbalance at the formation face, migration of this gas can occur to a lower pressure zone and possibly to the surface. It may occur during cementing operations i.e. before the cement set.

The causes of annular gas migration are varied:

- Flow through a channel created by incomplete mud removal or generated by excessive free water;
- A microannulus caused by thermal or pressure stresses placed on the casing during and after placement;
- Migration through the cement matrix during the early stages of hydration.

Gas migration through the cement matrix can occur due to the loss of hydrostatic pressure because of natural and unavoidable phenomena that occur during cement hydration. During the cement placement in the annulus, the slurry will develop at the beginning a low shear strength value that fully transmits the hydrostatic pressure. But when the gel strength development continues, the cement will not transmit the total hydrostatic pressure. Due to its capability to sustain shear stresses, pressure within the annulus is continuously reduced by slurry gelation. Gas migration can occur, resulting in a high risk of losing the control of the well.

Because the slurry gel strength development is the main cause of pressure decline in the cemented annulus, it has been quantified in different ways to predict a potentiality for gas migration. One of these consists in calculating:

The time period for the slurry to reach a gel strength value of 100 lb/100sq.ft: this period is called "zero gel time"
The time period for the slurry to increase its gel strength from 100 lb/100sq.ft to 500 lb/100sq.ft: this period is called "the transition time"

The former gel strength value of 100 lb/100sq.ft is related to the loss in hydrostatic pressure transmission: consequently for the zero gel time, the longer, the better. The later gel strength value of 500 lb/100sq.ft is related to the fact that it is considered that gas can not migrate anymore in slurry with such a gel strength value. This time period is considered as critical for gas migration and consequently, for transition time, the shorter the better. Consequently, the previously proposed approach is to attempt to achieve a slurry that has a long zero gel strength time and a short transition time in an attempt to ensure gas-tight behaviour. However, such slurries have not always proved to be gas-tight.

It is an object of the invention to provide improved methods for determining the gas migration behaviour of cement slurries to allow better gas-tight slurries to be designed.

In accordance with one aspect of the present invention, there is a method of determining the gas migration characteristics of a cement slurry, comprising measuring the shear modulus of the slurry over a period of time and using the measurement of the shear modulus to determine the gas migration characteristics of the slurry.

Preferably a series of shear modulus measurements are compared to corresponding gel strength measurements on the slurry. This comparison can comprise the ratio of the gel strength to the shear modulus. A slurry having a high shear modulus and a low ratio of gel strength to shear modulus (e.g. 0.03 - 0.04) is considered gas-tight. A slurry having a low shear modulus and a high ratio (e.g. 0.10 - 0.15) is considered as not gas-tight.

It is preferred that the measurements of shear modulus G' and gel strength GS are obtained using a rheometer having a rotor with blades (vanes) mounted thereon, the torque measured by the rheometer being used to calculate shear modulus according to

$$G' \text{ (Pa)} = \alpha * dT/d\Omega$$

$$\alpha = 4/(\pi D^3 * (2\text{-}2/B) * H/(0.5*D) + 1.56)$$

where:

    D = diameter of rotor
    H = height of rotor
    B = number of blades on the rotor

and gel strength according to

$$GS \text{ (lbf/ 100 sq.ft)} = \beta * Tm \text{ in (mN.m)}$$

$$\beta = 2/(\pi D^3 * (1/3 + H/D))$$

The invention will now be described by way of examples and in conjunction with the accompanying drawings, in which:

    Figure 1 shows a plot of torque vs. time for cement slurries A - D;
    Figure 2 shows a plot of shear modulus vs. gel strength for slurries A - D;
    Figure 3 shows an expanded section of Figure 2;
    Figure 4 shows a plot of gel strength development for Slurry A;
    Figure 5 shows a plot of gel strength development Slurry C; and
    Figures 6, 7 and 8 show plots of gel strength development for slurry B at 20, 60 and 120 minutes.

To determine the gas migration properties of a cement slurry, it is necessary to determine the rheological properties of the slurry (rheology is the study of the deformation and flow of fluids, it describes the flow pattern and physical properties of a fluid).

Viscosity is the internal resistance a fluid offers to flow due to the frictional force, which arises when one layer of fluid rubs against another. Considering a fluid as platelets moving parallel to one another at different velocities, for two platelets that are a small distance apart (r) and have equal areas (A), the frictional force (F) is known as the shear stress and is related to the relative speed ($V_1$, $V_2$) of the two platelets. The relative speed is known as the shear rate. Thus:

$$\text{Shear stress } (\tau) = F/A \text{ -dyne/cm}^2$$

$$\text{Shear rate } dv/dr = \gamma = (V_1 - V_2)/r \text{ -sec}^{-1}$$

$$\text{Viscosity } (\mu) = \text{shear stress/ shear rate -Pa.s}$$

The unit of measurement for viscosity ($\mu$) is the centipoise (cP), cP = mPa.sec.

The vane rheometer allows measurement of the gel strength and shear modulus of slurry samples. In principle, a vane rheometer is similar to a conditioner but the torque exerted on the vane to measure the gel strength is transmitted by means of a magnetic drive to avoid the need for a rotating mechanical seal that can generate unpredictable friction torque, and to ensure safe handling over the whole pressure and temperature range of the meter.

A small DC motor is fixed directly on the magnetic drive and measured by a force gauge measuring the reaction torque exerted by the gauge preventing rotation of the motor stator. To provide an appropriate working range and facilitate handling, the meter is made of two distinct parts, the cell assembly and the measuring head assembly, each of them including half of the magnetic drive. The measuring head is manually mounted on top of the cell assembly and magnetically locked by the magnetic drive to give the possibility to adapt the measuring head speed and torque capabilities without opening the cell.

For better performances the rotation speed range, two measuring heads can be provided. The first one rotates the vane at rotation speed covering the rotation speed (150rpm) recommended by the API to condition the slurry sample. The second one is designed to measure the resisting torque exerted by the slurry sample on the vane at very low rotation speed, typically 0.01rpm.

The vane itself is made of a spinner mounted within a cylinder. Small vanes are fixed on the external wall of the cylinder. At high speed, the vane acts as a spinner. The fluid is flowing down inside the vane cylinder and flowing up in the annulus between the vane and the cell wall. The fluid recirculation created within the cell ensures the proper conditioning of the slurry. Reverse circulation is also possible. The described circulation is preferred as it prevents the contamination of the bearings supporting the vane rotation axis resulting in unwanted friction torque. At low rotation speed, the spinner becomes inefficient and the vane reacts as a standard vane.

The rotation speed and the test parameters (pressure temperature and torque) are controlled and recorded by the meter electronic controller.

Slurries are mixed according to the API procedure. The slurry is first conditioned at 150 rpm with the high-rotation-speed-measuring-head during 20 minutes. The temperature in the cell is controlled by a heating jacket coupled with a temperature regulator, which can keep the temperature at 160deg.F. The six -blade vane is connected via its axis to a torque head. After the conditioning, the high-rotation-speed-head is replaced by the low-rotation-speed-head without opening the cell to maintain the slurry in the test conditions. The gel strength development measurement is performed at regular interval of time (10 minutes) at low rotation speed (0.01 RPM) to avoid shocks, which could break the gel. The torque is measured every 1/10$^e$ of a second over the 99 seconds of the rotation time. Then the motor is switched off and the slurry left static until the next measurement. The system automatically detects the torque values during each period and memorises the entire gel strength development curve.

For each measuring point (every 10 minutes), Static Gel Strength (SGS) in Pa and Shear Modulus G' (Pa) are determined from maximum torque value Tm (mNm) and the slope dT/d$\Omega$ (mNm/rd) respectively using the appropriate equations.

The maximum value of the torque (Tm) allows the computation of the Gel Strength (GS) using the following equation 1:

$$\text{GS \{lbf/100sq.ft\}} = \beta * \text{Tm in (mN.m)} \qquad [1]$$

$$\beta = 2/(\pi D^3 * (1/3 + H/D))$$

The maximum value of the torque also allows the computation of the shear modulus (G') using equation 2:

$$\text{G' (Pa)} = \alpha * dT/d\Omega \qquad [2]$$

$$\alpha = 4/(\pi D^3 * (2-2/B) * H/(0.5*D) + 1.56$$

For the large vane:

Diameter (D) = 64 mm
Height (H) = 68 mm
Blade number (B) = 6

A series of tests are performed using the rheometer described above on various slurries. These slurries include fluid-loss control additives or existing gas migration control additives together with other additives to modify the behaviour of the slurry. The additives used in these slurries are described below. For each slurry, the rheology and the free water are measured. All the slurries tested with the vane rheometer are optimized at 160°F to reach a given dispersion state and a good stability (no free water, no settling). In order to be able to compare the gel strength development of the slurries, the slurries are optimized to obtain a given dispersion state (Ty =6 +/- 3 lb/100 sq.ft) after conditioning using the appropriate dispersant concentration.

Four cement slurries are tested to identify properties indicating a gas-tight or non-gast-tight slurry.

Slurry A includes a fluid loss/gas migration control additive comprising a latex additive (latices are aqueous dispersions of solid polymer particles, including surfactants and protective colloids, which impart stability to the dispersion). It contains about 50% insoluble particles, with a particle size of about 0.30 micron.

Slurry B includes a fluid loss/gas migration control additive comprising a suspension of polymeric microgel particles that provide gas migration control to cement slurries. Particle size is slightly below 70 nanometers and particle concentration is about 50%.

Slurries A and B are examples of gas-tight slurries.

Slurry C includes a solid cement additive that provides fluid loss control. It is a powder that can be dissolved in the mix water with the cement and comprises a high molecular weight, synthetic water-soluble polymer with fluid loss control properties.

Slurry D includes a fluid-loss control additive comprising a non-retarding liquid that can be used in fresh water slurries. It is a water solution of synthetic polymer, which is readily dispersed in the mix water to provide uniform distribution throughout the cement system.

Slurries C and D are examples of non-gas-tight slurries.

The table below shows the composition of the slurries used in the tests and the basic rheology at BHCT (BHCT = Bottom Hole Circulating Temperature, i.e. the temperature encountered by the cement slurry when being pumped into the well, in this case 160°F).

| Design | **Slurry A** | **Slurry B** | **Slurry C** | **Slurry D** |
|---|---|---|---|---|
| Liquid Additive gps | 1 | 1.5 | - | 0.4 |
| Liquid Dispersant gps | 0.06 | 0.076 | - | 0.06 |
| Solid Additive %BWOC | - | - | 0.3 3 | - |
| Solid Dispersant %BWOC | - | - | 0.3 | - |
| Antifoam gps | 0.03 | 0.03 | 0.03 | 0.03 |
| Fluid loss API (ml) | 36 | 40 | 70 | 75 |

| **Rheology at BHCT** | | | | |
|---|---|---|---|---|
| PV (cp) | 18 | 44 | 63 | 93 |
| Ty (1b/100sqft) | 4 | 7 | 7 | 9 |
| Free water | 0.5 | 0.8 | 0 | 0 |

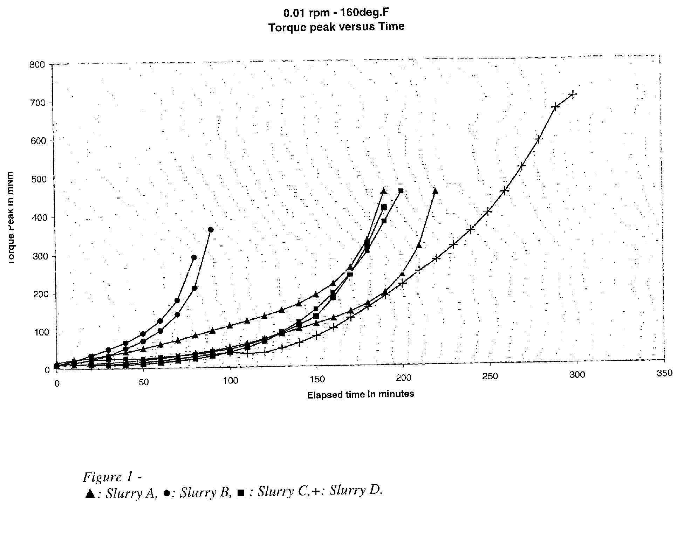

Figure 1 shows a plot of rheometer torque measurement vs. time for slurries A - D, which are used for the determination of zero gel time and transition time in accordance with the prior art method of determining gas migration properties. As can be seen, Slurry B appears not to have the properties of a gas-tight cement whereas Slurries C and D do. The behaviour of Slurry A is not particularly clear in this plot.

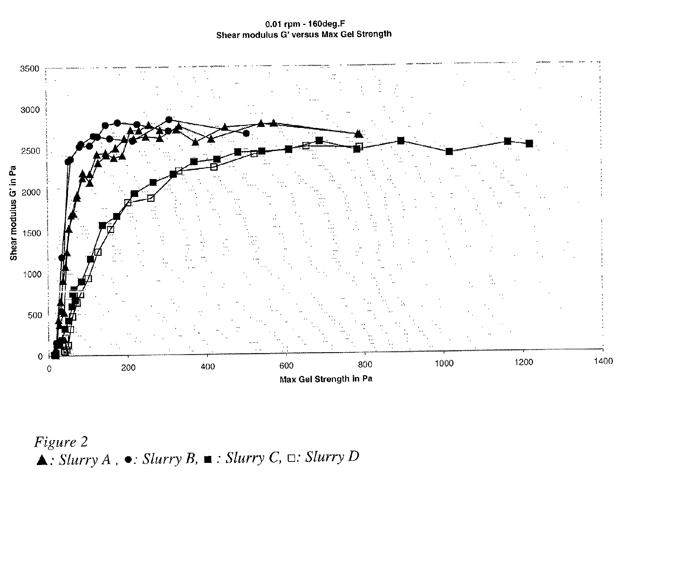

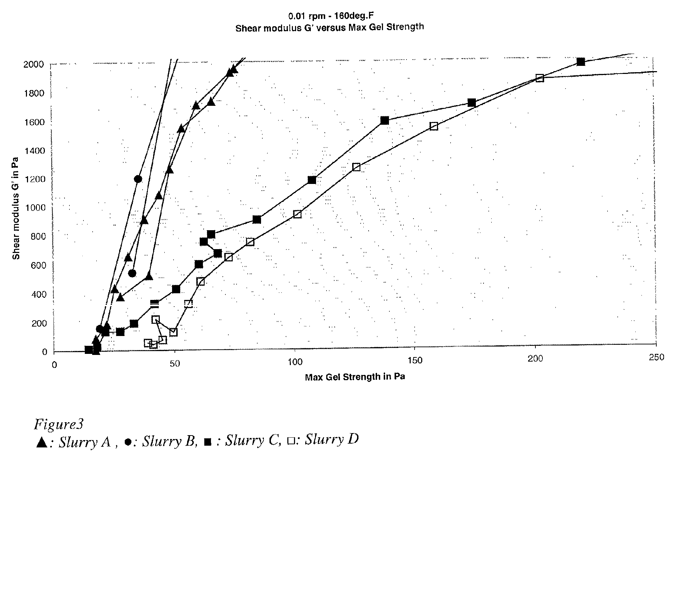

Figure 2 shows a plot of shear modulus G' vs. gel strength. Figure 3 is an expanded version of the early part of this plot. The two families of slurries: gas-tight (Slurries A and B) and non-gas-tight (Slurries C and D) are clearly differentiated by this method.

The maximum shear modulus of 2500 Pa seen in Figure 2 is a mechanical limitation of the vane rheometer. Because of restriction of magnetic transmission in the head, it is not possible to measure above this limit but it is possible to avoid this problem by modification of the measurement head. For the different behaviour of the slurries, it is useful to look at the first part of the curves until the plateau. The ratio SGS/ G' allows to discriminate two families: Gastight family and non Gastight family.

Figure 3 presents the result of the measurement of the Shear modulus G'and Max Gel Strength but zooming in on period before reaching the plateau. The ratios of Slurries C and D are in the same range respectively 0.11 and 0.10. The ratio of Slurry A is 0.04 and of Slurry B 1.5 gps the ratio is 0.032. The two different families can be distingished by their ratio. The slurries containing gas-migration control additives have a lower ratio than the slurries with conventional fluid-loss control additives.

The migration gas prevention is better with slurries developing high shear modulus G'. It is possible to differentiate two families:

- the gastight family with a high shear modulus and a low ratio or low deformation at rupture.
- the no gastight family with a low shear modulus and a high ratio.

During the tests a difference is noticed between the slurries containing a gastight additive and the ones containing only conventional fluid-loss control additives. When measuring the torque versus time during 99 seconds, slurry can act in two different ways:

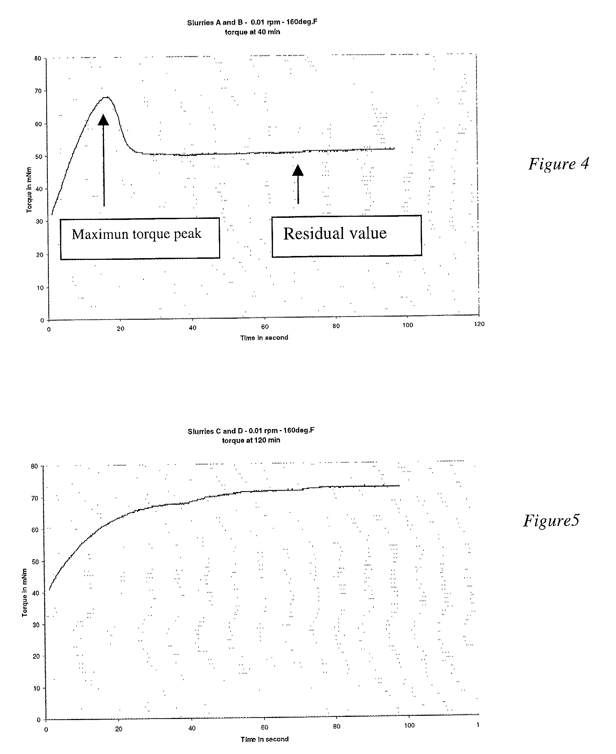

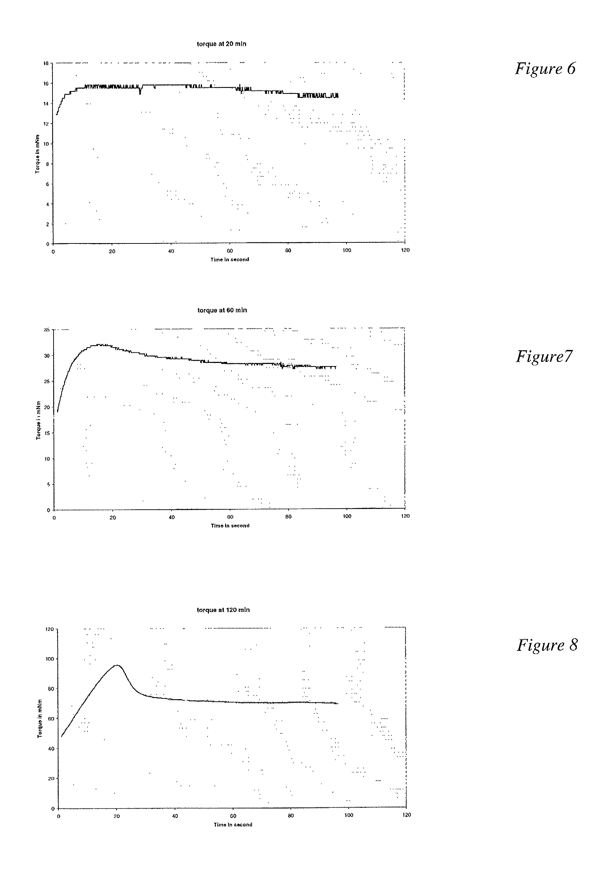

- Fragile as seen in figure 4
- Ductile as seen in figure 5

In case of fragile behaviour as seen in figure 4 the static gel strength breaks before 20 seconds by turning the vane cylinder during 99 seconds. This behaviour is noticed with both Slurries A and B. The possibility to break the gel strength during a field placement of a slurry appears when the casing shows little movements during the cement placement.

Figure 5 shows the behaviour of non-gastight slurry C. It is not possible to break the gel formed during the placement by turning the vane. The ductile behaviour of Slurry C stays the same during the whole measurement. Slurry shows the same behaviour.

With Slurry B a difference is noticed in the time in which the transition from the non- fragile to fragile behaviour appears. The figures 6, 7 and 8 show the gel strength development of Slurry B at respectively 20, 60 and 120 minutes.

By measuring the shear modulus and gel strength in their manner described, it is possible to identify gas-tight slurries and analyse the effects of various additives when designing a slurry for use in a situation in which gas migration might be a problem.

**Claims**

1. A method of determining the gas migration characteristics of a cement slurry, comprising measuring the shear modulus and gel strength of the slurry over a period of time and comparing the shear modulus and gel strength measurements to determine the gas migration characteristics of the slurry.

2. A method as claimed in claim 1, wherein the step of comparing comprises determining the ratio of the gel strength to the shear modulus.

3. A method as claimed in claim 2, comprising comparing the shear modulus and a ratio of gel strength to shear modulus to determine the gas migration characteristics of the slurry.

4. A method as claimed in claim 3, comprising characterising a slurry as gas tight if it has a high shear modulus and a low ratio of gel strength to shear modulus and a low deformation at the rupture, and as not gas tight if it has a low shear modulus and a high ratio of gel strength to shear modulus.

5. A method as claimed in claim 4, wherein the low ratio has a value of about 0.03 - 0.04 and the high ratio has a value of about 0.10-0.15.

6. A method as claimed in any preceding claim, wherein the measurements of shear modulus G' and gel strength GS are obtained using a rheometer having a rotor with blades mounted thereon, the measurement comprising measuring the torque on the rotor when stirring a sample of the slurry.

7.  A method as claimed in claim 6, wherein the shear modulus is determined according to:

$$G' \text{ (Pa)} = \alpha * dT/d\Omega$$

$$\alpha = 4/(\pi D^3 * (2\text{-}2/B) * H/(0.5*D) + 1.56)$$

where:

D = diameter of rotor
H = height of rotor
B = number of blades on the rotor.

8.  A method as claimed in claim 6 or 7, wherein the gel strength is determined according to

$$GS \text{ (lbf/100 sq.ft)} = \beta * Tm \text{ in (mN.m)}$$

$$\beta = 2/(\pi D^3 * (1/3 + H/D)).$$

**0.01 rpm - 160deg.F**
**Torque peak versus Time**

*Figure 1 -*
*▲: Slurry A, ●: Slurry B, ■ : Slurry C,+: Slurry D.*

0.01 rpm - 160deg.F
Shear modulus G' versus Max Gel Strength

*Figure 2*
▲ : *Slurry A* , ● : *Slurry B,* ■ : *Slurry C,* □ : *Slurry D*

**0.01 rpm - 160deg.F**
**Shear modulus G' versus Max Gel Strength**

*Figure3*
▲ : *Slurry A ,* ● : *Slurry B,* ■ : *Slurry C,* □ : *Slurry D*

Slurries A and B - 0.01 rpm - 160deg.F
torque at 40 min

*Figure 4*

Slurries C and D - 0.01 rpm - 160deg.F
torque at 120 min

*Figure5*

torque at 20 min

*Figure 6*

torque at 60 min

*Figure7*

torque at 120 min

*Figure 8*

EP 1 298 432 A1

**EUROPEAN SEARCH REPORT**

Application Number

EP 01 40 2502

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | KHALID A. AL-BURAIK, KHALID A. AL-ABDULQADER, RIAD BSAIBES: "Prevention of Shallow Gas Migration Through Cement" 'Online! 1998 , SAUDI ARAMCO JOURNAL OF TECHNOLOGY XP002184031 Retrieved from the Internet: <URL: http://www.saudiaramco.com/publications/jot/Fall1998/6jot_fall1998.pdf> 'retrieved on 2001-11-23! * page 37, paragraph 2 * | 1-8 | G01N33/38 |
| X | ANDREW K. WOJTANOWICZ, JOHN ROGERS SMITH, WOJCIECH M. MANOWSKI, JOSEPH MARTIN: "Top Cement Pulsation for Prevention of Flow after Cementing" 'Online! 15 September 2000 (2000-09-15) , LOUISIANA STATE UNIVERSITY , BATON ROUGE, LOUISIANA XP002184032 Retrieved from the Internet: <URL: http://www.bourgoyneenterprises.com/lsu/mms/30749/Top%20Cement%20Pulsation.pdf> 'retrieved on 2001-11-23! * page 2, paragraph 2 * * page 12, paragraph 1 - page 14, paragraph 2; figure 10 * | 1-8 | |
| | | | TECHNICAL FIELDS SEARCHED (Int.Cl.7) |
| | | | G01N |
| X | EP 0 538 989 A (HALLIBURTON CO) 28 April 1993 (1993-04-28) * page 4, line 57 - page 5, line 5; example 1 * | 1-8 | |
| X | US 6 171 386 B1 (SABINS FREDDIE LYNNE) 9 January 2001 (2001-01-09) * column 1, line 65 - column 2, line 24; figures 1,2 * * column 10, line 44 - line 47 * | 1-8 | |
| | -/-- | | |

The present search report has been drawn up for all claims

| Place of search | Date of completion of the search | Examiner |
|---|---|---|
| MUNICH | 26 November 2001 | Jochheim, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

## EUROPEAN SEARCH REPORT

Application Number

EP 01 40 2502

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | "Cement Gas Migration Analyzer Model 7150" 'Online! 1997 , CHANDLER ENGINEERING , TULSA, OKLAHOMA XP002184033 Retrieved from the Internet: <URL: http://www.chandlerengineering.com/sales_literature/7150.pdf> 'retrieved on 2001-11-23! * the whole document * | 1-8 | |
| | | | **TECHNICAL FIELDS SEARCHED (Int.Cl.7)** |

The present search report has been drawn up for all claims

| Place of search | Date of completion of the search | Examiner |
|---|---|---|
| MUNICH | 26 November 2001 | Jochheim, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 01 40 2502

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2001

| Patent document cited in search report | | Publication date | Patent family member(s) | | Publication date |
|---|---|---|---|---|---|
| EP 0538989 | A | 28-04-1993 | US | 5149370 A | 22-09-1992 |
| | | | DE | 69210941 D1 | 27-06-1996 |
| | | | DE | 69210941 T2 | 02-10-1996 |
| | | | EP | 0538989 A2 | 28-04-1993 |
| | | | NO | 305238 B1 | 26-04-1999 |
| US 6171386 | B1 | 09-01-2001 | NONE | | |